(19) **Europäisches Patentamt**

**European Patent Office**

**Office européen des brevets**

(11) **EP 0 650 344 B1**

(12) **EUROPÄISCHE PATENTSCHRIFT**

(45) Veröffentlichungstag und Bekanntmachung des Hinweises auf die Patenterteilung:
**18.02.1998  Patentblatt 1998/08**

(21) Anmeldenummer: 93911717.2

(22) Anmeldetag: **11.05.1993**

(51) Int. Cl.$^6$: **A61B 17/58**

(86) Internationale Anmeldenummer:
**PCT/CH93/00118**

(87) Internationale Veröffentlichungsnummer:
**WO 94/26190 (24.11.1994  Gazette 1994/26)**

(54) **OSTEOSYNTHETISCHES BEFESTIGUNGSELEMENT UND MANIPULIERHILFE DAZU**

OSTEO-SYNTHETIC SECURING COMPONENT AND MANIPULATION AID THEREFOR

ELEMENT DE FIXATION POUR OSTEOSYNTHESE ET ACCESSOIRE DE MANIPULATION

(84) Benannte Vertragsstaaten:
**CH DE ES FR GB IT LI**

(43) Veröffentlichungstag der Anmeldung:
**03.05.1995  Patentblatt 1995/18**

(73) Patentinhaber: **Synthes AG, Chur
7002 Chur (CH)**

(72) Erfinder:
• **SCHLÄPFER, Johannes, Fridolin
CH-8750 Glarus (CH)**
• **FRIGG, Robert
CH-7270 Davos Platz (CH)**
• **AMREIN, Thomas
CH-6048 Horw (CH)**

• **RECHER, Daniel
CH-4434 Höllstein (CH)**
• **TREBING, Linda
Devon, PA 19333 (US)**

(74) Vertreter:
**Lusuardi, Werther Giovanni, Dr.
Dr. Lusuardi AG,
Kreuzbühlstrasse 8
8008 Zürich (CH)**

(56) Entgegenhaltungen:
**EP-A- 0 465 158          EP-A- 0 528 177
EP-A- 0 535 623          FR-A- 2 659 225**

## Beschreibung

Die Erfindung bezieht sich auf ein osteosynthetisches Befestigungselement, insbesondere eine Pedikelschraube oder einen Wirbelsäulenhaken gemäss der Gattung des Patentanspruchs 1 sowie eine Vorrichtung zur Manipulation des osteosynthetischen Befestigungselementes.

Aus der DE-U1 89.15.443.6 ist bereits ein Befestigungselement dieser Gattung, insbesondere für die Wirbelsäulenchirurgie bekannt. Es besteht im wesentlichen aus einem am Knochen verankerbaren, unteren Teil in Form eines Schraubenschaftes oder einer Klinge und einem daran anschliessenden, oberen Körper zur Befestigung an einer Stange, wobei in dem Körper ein nach oben mündender Kanal ausgebildet ist, der zwei seitliche Schenkel begrenzt, zwischen denen die Stange aufgenommen werden kann. Die Fixation der Stange innerhalb des Kanals erfolgt durch einen von oben zwischen die Schenkel einschraubbaren Gewindestopfen, dessen unteres, zur Anlage an die Stange bestimmtes Ende mit Verhakungsmitteln in Form einer oder mehrerer Spitzen versehen ist.

Nachteilig bei diesem Implantat ist seine schlechte Manipulierbarkeit während des Anziehens, was oft dazu führt, dass der Längsträger nicht optimal geklemmt ist und mit der Zeit aus der Schraube rutschen kann.

Aus der EP 465.158 MEHDIAN ist eine Pedikelschraube gemäß den Oberbegriffen der unabhängigen Ansprüche 1, 10 und 11 mit einem Durchgangskanal zur Aufnahme eines Langsträgers bekannt, welche einen rotationsstabilen, lösbaren Verbindungsmechanismus zur form- und kraftschlüssigen, temporaren Aufnahme einer Manipulationsvorrichtung aufweist. Nachteilig bei dieser bekannten Pedikelschraube ist allerdings die Tatsache, dass der Verbindungsmechanismus mit dem Durchgangskanal interferiert.

Hier will die Erfindung Abhilfe schaffen. Der Erfindung liegt die Aufgabe zugrunde ein osteosynthetisches Befestigungselement und eine Vorrichtung zur Manipulation dieses osteosynthetischen Befestigungselementes zu schaffen, welche einerseits eine kraftvolle intraoperative Manipulation des Implantates gestatten und je nach Ausführungsform des Implantates die Möglichkeit bieten, das Befestigungselement simultan mit dem Manipulationsvorgang in seiner relativen Lage zum Knochen, bzw. zu anderen Befestigungselementen anzuziehen.

Die Erfindung löst die gestellte Aufgabe mit einem osteosynthetischen Befestigungselement, welches die Merkmale des Anspruchs 1, 10 oder 11 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass damit ein kraftvolles Ein- und Ausdrehen von Pedikelschrauben in den Wirbelknochen und ein sicheres Ein-, bzw. Aushaken von Wirbelsäulenhaken ermöglicht wird. Wenn die Pedikelschraube einmal in den Knochen eingedreht ist (bzw. der Wirbelsäulenhaken eingehängt ist), kann die Manipulierhilfe dazu benutzt werden, um über die Schraube (bzw. den Haken) Kräfte und Momente auf die Wirbelsäule auszuüben und dadurch Deformitäten zu manipulieren. Weitere Vorteile bestehen darin, dass die Manipulierhilfe jederzeit entfernt und wieder montiert werden kann und dass mittels der Manipulierhilfe das Implantat derart bewegt werden kann, dass der Längsträger optimal zum Implantat zu liegen kommt.

Die Erfindung und Weiterbildungen der Erfindung werden im folgenden anhand der teilweise schematischen Darstellungen mehrerer Ausführungsbeispiele noch näher erläutert.

Es zeigen:

Fig. 1 eine perspektivische Darstellung des osteosynthetischen Befestigungselement in Form einer Pedikelschraube;

Fig. 2 eine perspektivische Darstellung des osteosynthetischen Befestigungselement in Form eines Wirbelhakens;

Fig. 3 einen Querschnitt durch den oberen Teil der Pedikelschraube nach Fig. 1;

Fig. 4 eine explosionsartige Darstellung der erfindungsgemässen Manipulationsvorrichtung;

Fig. 5 eine perspektivische Darstellung der zusammengesetzten Manipulationsvorrichtung nach Fig. 4;

Fig. 6 - 12 perspektivische Darstellungen weiterer Ausführungsformen des erfindungsgemässen Befestigungselementes;

Fig. 13 einen Querschnitt durch den oberen Teil einer Ausführungsform des erfindungsgemässen Befestigungselementes und des unteren Teils der Manipulationsvorrichtung; und

Fig. 14 eine perspektivische Darstellung einer weiteren Variante eines Befestigungselementes.

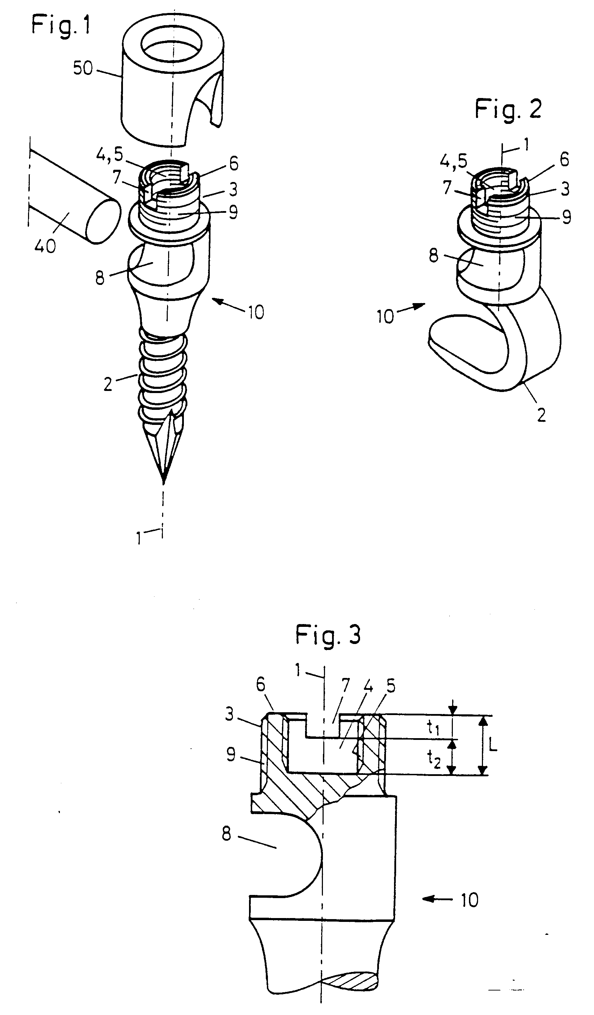

Das in Fig. 1, bzw. 2 in Form einer Pedikelschraube, bzw. eines Wirbelhakens dargestellte erfindungsgemässe Befestigungselement 10 besteht im wesentlichen aus einem am Knochen verankerbaren unteren Abschnitt 2 (Schraubenschaft oder Hakenklinge) und einem in Richtung seiner Längsachse 1 daran anschliessenden, oberen Abschnitt 3, welcher von einem quer zur Längsachse 1 verlaufenden Durchgangskanal 8 zur Aufnahme eines Längsträgers 40 vollständig durchquert ist und welcher mit einem Aussengewinde 9 versehen ist, um einen, den Längsträger 40 blockierenden Verschlussteil 50 aufzunehmen.

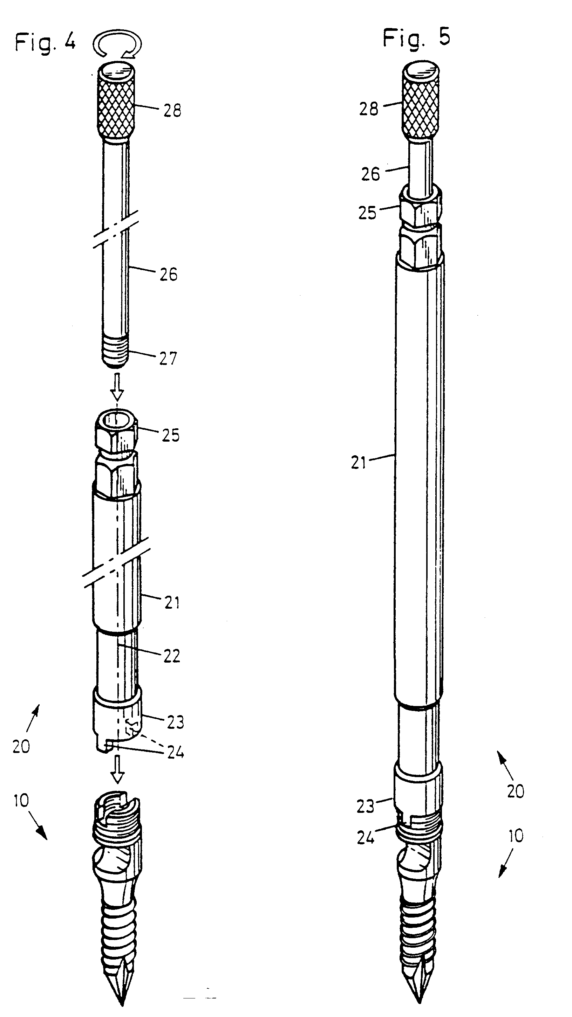

Wie in Fig. 3 dargestellt ist der obere Abschnitt 3 an seinem oberen Ende 6 mit einem rotationsstabilen, lösbaren Verbindungsmechanismus 4,5,7 versehen, zur form- und kraftschlüssigen, temporären Aufnahme einer Manipulationsvorrichtung 20. Der Verbindungsmechanismus 4,5,7 besteht aus einer in Richtung der Längsachse 1 verlaufenden, kreiszylindrischen, nach oben offenen Bohrung 4 mit einem Innengewinde 5 sowie einem quer zur Längsachse 1 verlaufenden Schlitz 7 der Tiefe $t_1$, der die formschlüssige Aufnahme einer Manipulationsvorrichtung 20 (Fig. 4) mit zwei entsprechenden Nocken 24 gestattet. Der Schlitz 7 dient zur Rotationsstabilität. Das Innengewinde 5 kann mehrgängig ausgeführt werden um den Kupplungsvorgang zeitlich stark zu kürzen.

Die Bohrung 4 mit ihrem Innengewinde 5 setzt sich vom Boden des Schlitzes 7 um den Betrag $t_2$ in Richtung des unteren Abschnitts 2, zur kraftschlüssigen Aufnahme einer Manipulationsvorrichtung 20 mit einem entsprechenden Aussengewinde 27 (Fig. 4) fort. Dabei weist die Bohrung 4 mindestens die Länge $L = t_1 + t_2$ auf und gestattet eine rotationsstabile, lösbare Verbindung mit der Manipulationsvorrichtung 20.

Die in Fig. 4 dargestellte Manipulationsvorrichtung 20 besteht aus einer hohlzylindrischen Hülse 21 mit der Längsachse 22, die an ihrem einen Ende 23 eine quer zur Längsachse 22 verlaufenden Nocken 24 aufweist. Vom anderen Ende 25 der Hülse 21 ist ein zylindrischer Stift 26 in die Hülse 21 einführbar. Der zylindrische Stift 26 trägt an seinem einen Ende ein Aussengewinde 27, welches - nach erfolgter Einführung - über das Ende 23 der Hülse 21 hervorragt und an seinem anderen Ende ein zum Anziehen der Verbindung dienendes, aus der Hülse 21 vorstehendes Griffende 28 aufweist.

Wie in Fig. 5 gezeigt, kann die an das Befestigungselement 10 gekoppelte Manipulationsvorrichtung 20 zur Übertragung von Zug- und Druckkräften sowie von Momenten auf das Befestigungselement 10 eingesetzt werden.

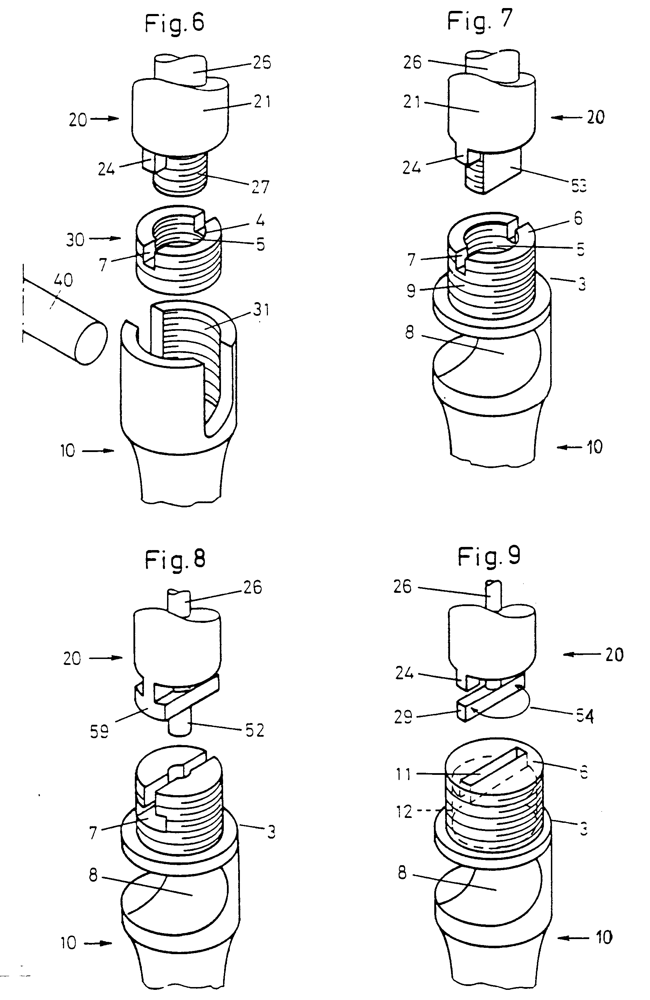

Fig. 6 zeigt eine Variante des Befestigungselementes 10, bei welchem der obere Abschnitt 3 zweiteilig ausgeführt ist, derart, dass der aus Bohrung 4, Innengewinde 5 und Schlitz 7 bestehende Verbindungsmechanismus 4,5,7 als in das Innengewinde 31 des oberen Abschnitts 3 einschraubbarer, als Gewindestopfen ausgebildeter Verschlussteil 30 realisiert ist. Die Manipulationsvorrichtung 20 wird dann mit diesem separaten Verschlussteil 30 über das Innengewinde 5 gekoppelt, um damit das Befestigungselementes 10 manipulieren und gleichzeitig das Verschlussteil 30 im Innengewinde 31 anziehen zu können.

In Fig. 7 ist eine weitere Variante des Kupplungsprinzips zwischen Befestigungselement 10 und Manipulationsvorrichtung 20 dargestellt, welche analog zu einem Bajonettverschluss funktioniert. Am Ende des Stiftes 26 der Manipulationsvorrichtung 20 ist, statt des vollen Gewindes 27, ein beidseitig angeflachter Gewindebolzen 53 vorgesehen, der nach Einschiebung in den Schlitz 7 um 90° gedreht und damit verriegelt wird.

In Fig. 8 ist eine weitere Variante des Kupplungsprinzips zwischen Befestigungselement 10 und Manipulationsvorrichtung 20 dargestellt, wobei das Ende des Stiftes 26 der Manipulationsvorrichtung 20 als T-Stück 59 mit einem Stift 52 ausgebildet ist. Analog dazu ist der Schlitz 7 des Befestigungselementes 10 T-förmig ausgebildet. Das T-Stück 59 wird zur Kupplung seitlich in den Schlitz 7 eingefahren und der Stift 52 nach unten gestossen um ein Herausgleiten aus dem Schlitz 7 zu verhindern. Dieses konstruktive Prinzip kann auch invertiert werden, indem der obere Abschnitt 3 an seinem oberen Ende 6 T-förmig und statt dessen die Manipulationsvorrichtung 20 hufeisenförmig ausgebildet wird.

In Fig. 9 ist ein eigentlicher Bajonettverschluss zwischen dem Befestigungselement 10 und der Manipulationsvorrichtung 20 dargestellt. Am Ende des Stiftes 26 ist ein T-förmiges Element 29 vorgesehen und das obere Ende 6 des oberen Abschnitts 3 weist einen Schlitz 11 auf, der sich nach unten zu einem kreiszylinderförmigen Hohlraum 12 erweitert. Das T-förmige Element 29 lässt sich somit durch den Schlitz 11 in den kreiszylinderförmigen Hohlraum 12 einführen und kann dort um 90° verdreht werden. Die Nocken 24 werden dabei lediglich wie ein Schraubenzieher in den Schlitz 11 eingeführt. Das T-förmige Element 29 erlaubt das Aufbringen von Zug und Druck auf das Befestigungselement 10, währenddem die Nocken 24 das Vor- und Zurückschrauben des Befestigungselementes 10 in Richtung des Pfeiles 54 gestatten.

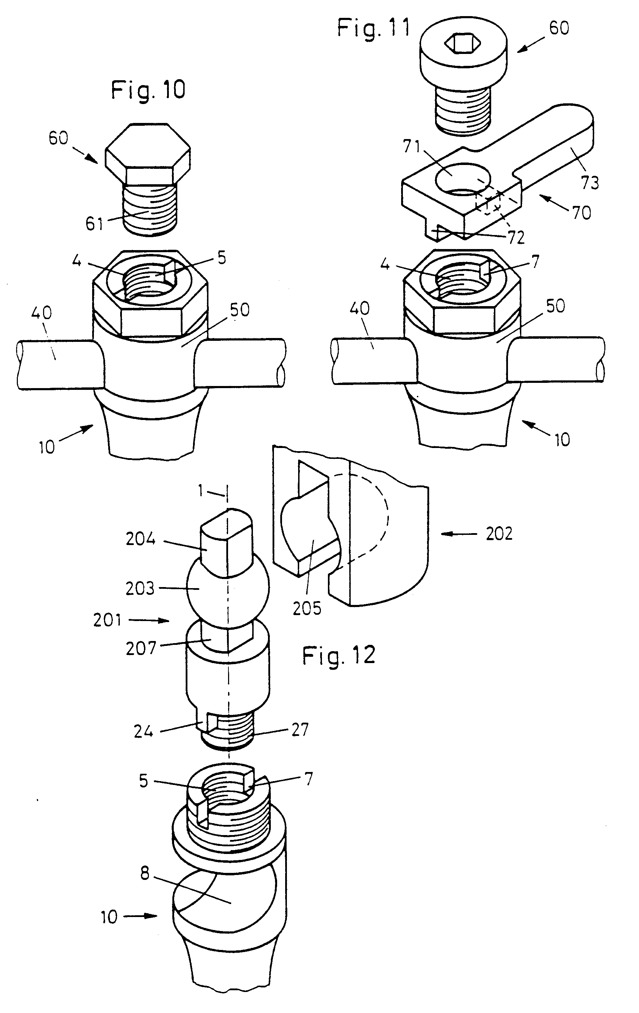

Die in den Fig. 10 und 11 gezeigten Beispiele zeigen die Bedeutung des Innengewindes 5 für die Befestigung von Elementen, die nicht in direktem Zusammenhang mit der Manipulierhilfe stehen.

In Fig. 10 ist ein Befestigungselement 10 dargestellt, bei welchem zusätzlich eine Schraube 60 mit einem, mit dem Innengewinde 5 korrespondierenden Aussengewinde 61 vorgesehen ist, welche bis in den Bereich des Durchgangskanals 8 einschraubbar ist, so dass damit der Längsträger 40 fixierbar ist. Auf diese Weise kann die Festigkeit der Verbindung erheblich erhöht werden.

In Fig. 11 ist ein Befestigungselement 10 dargestellt, bei welchem zusätzlich ein Querkörper 70 vorgesehen ist, der eine mit der Bohrung 4 korrespondierende Öffnung 71, zwei radial gegenüberliegende, mit dem Schlitz 7 korrespondierende Nocken 72 sowie einen radial von der Öffnung 71 sich wegerstreckenden Querfortsatz 73 zur Verbindung mit anderen Befestigungslementen 10 aufweist und welcher mittels einer Schraube 60 am oberen Abschnitt 3 befestigbar ist.

In Fig. 12 ist eine Variante der Manipulationsvorrichtung 20 dargestellt, welche aus zwei miteinander gelenkig verbindbaren Teilen besteht, wovon der eine Teil 201 den Kupplungsteil 24,27 umfasst und der andere Teil 202 Bestandteil eines weiteren Instrumentes, vorzugsweise eine Zange zum Reponieren von Wirbeldeformitäten, ist. Der eine Teil 201 weist einen kugelförmigen Teil 203 mit einem zylinderförmigen Fortsatz 204 auf, der mit einer entsprechenden seitlichen Öffnung 205 im anderen Teil 202 der Vorrichtung 20 korrespondiert. Das Verbindungsstück 207 ist beidseitig angeflacht um eine Rotation des Befestigungselementes 10 relativ zum Instrument 202 um die Längsachse 1 des Befestigungslementen 10 zu vermeiden.

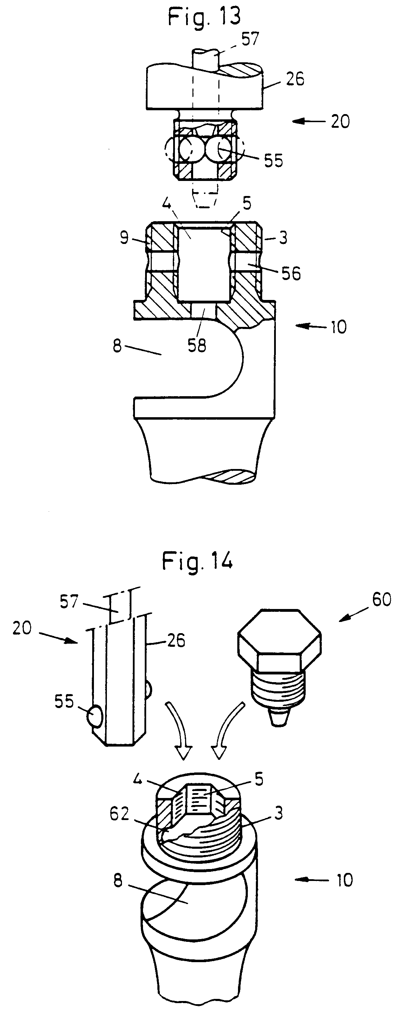

In Fig. 13 weist der obere Teil 3 des Befestigungselementes 10 mindestens eine Querbohrung 56 auf. Entsprechend dazu sind Kugeln 55 im Stift 26 der Manipulationsvorrichtung 20 vorgesehen. Wenn die Manipulationsvorrichtung 20 in die Bohrung 4 eingeführt ist und die Kugeln 55 mit den Querbohrungen 56 übereinstimmen, kann ein Stift 57 durch die zentrale Bohrung der Manipulationsvorrichtung 20 geschoben werden, wobei der Stift 57 die Kugeln 55 in die Querbohrungen 56 drückt. Damit ist es möglich mittels der Manipulationsvorrichtung 20 Zug-, Druck- und Rotationskräfte zu übertragen. Die Bohrung 58 dient zur Aufnahme des stiftartigen Endes der Sicherheitsschraube 60.

Schliesslich ist in Fig. 14 ein Befestigungselement 10 mit einer als Innensechskant ausgestalteten Bohrung 4 dargestellt, welche mit einem Hinterstich 62 für die Kugeln 55 der Manipulationsvorrichtung 20 versehen ist. Die Kugeln 55 werden analog zur Ausführungsform nach Fig. 13 mittels des Stiftes 57 in die Hinterlöcher 62 gedrückt. Der Innensechskant weist in den Flanken ein Innengewinde 5 zur Befestigung weiterer Implantate auf, wie z.B. der Sicherheitsschraube 60.

**Patentansprüche**

1. Osteosynthetisches Befestigungselement, insbesondere eine Pedikelschraube oder ein Wirbelsäulenhaken, mit einem Verschlussteil (30;50), mit einer Längsachse (1), mit einem am Knochen verankerbaren unteren Abschnitt (2) und einem in Richtung der Längsachse (1) daran anschliessenden, oberen Abschnitt (3), welcher an seinem unteren Ende von einem quer zur Längsachse (1) verlaufenden Durchgangskanal (8) zur Aufnahme eines Längsträgers (40) vollständig durchquert ist und welcher mit einem Innengewinde (5;31) und/oder einem Aussengewinde (9) versehen ist, um den den Längsträger (40) blockierenden Verschlussteil (30;50) aufzunehmen, wobei der obere Abschnitt (3) an seinem oberen Ende (6) oder an dem daran eingesetzten Verschlussteil (30;50) mit einem rotationsstabilen, lösbaren Verbindungsmechanismus (4,5,7;11,12) zur form- und kraftschlüssigen, temporären Aufnahme einer Manipulationsvorrichtung (20) versehen ist,
**dadurch gekennzeichnet, dass**
der obere Abschnitt (3) an seinem oberen Ende (6) oder das Verschlussteil (30;50) einen quer zur Längsachse (1) verlaufenden Schlitz (7) der Tiefe $t_1$, zur Aufnahme der Manipulationsvorrichtung (20) mit entsprechenden Nocken (24), aufweist.

2. Befestigungselement nach Anspruch 1, dadurch gekennzeichnet, dass

der obere Abschitt (3) eine in Richtung der Längsachse (1) verlaufende, kreiszylindrische, nach oben offene Bohrung (4) mit einem Innengewinde (5) aufweist, wobei
die Bohrung (4) mit ihrem Innengewinde (5) sich vom Boden des Schlitzes (7) um den Betrag $t_2$ in Richtung des unteren Abschnitts (2), zur kraft- und/oder formschlüssigen Aufnahme einer Manipulationsvorrichtung (20) mit einem entsprechenden Aussengewinde (27), fortsetzt; und
die Bohrung (4) mindestens die Länge $L = t_1 + t_2$ aufweist und eine rotationsstabile, lösbare Verbindung mit einer Manipulationsvorrichtung (20) gestattet.

3. Befestigungselement nach Anspruch 1, dadurch gekennzeichnet, dass der Verbindungsmechanismus des oberen Abschnitts (3) aus einem Bajonettverschluss besteht.

4. Befestigungselement nach Anspruch 3, dadurch gekennzeichnet, dass der Bajonettverschluss aus einem Schlitz (11) im oberen Abschnitt (3) besteht, der vorzugsweise eine zentrale Bohrung mit Innengewinde aufweist.

5. Befestigungselement nach Anspruch 2, dadurch gekennzeichnet, dass der obere Abschnitt (3) zweiteilig ausgeführt ist, derart, dass der aus Bohrung (4), Innengewinde (5) und Schlitz (7) bestehende Verbindungsmechanismus (4,5,7) als in das Innengewinde (31) des oberen Abschnitts (3) einschraubbarer, als Gewindestopfen ausgebildeter Verschlussteil (30) realisiert ist.

6. Befestigungselement nach einem der Ansprüche 1 - 5, dadurch gekennzeichnet, dass zusätzlich eine Schraube (60) mit einem, mit dem Innengewinde (5) korrespondierenden Aussengewinde (61) vorgesehen ist, welche bis in den Bereich des Durchgangskanals (8) einschraubbar ist, so dass damit der Längsträger (40) fixierbar ist.

7. Befestigungselement nach einem der Ansprüche 2, 5 oder 6, dadurch gekennzeichnet, dass zusätzlich ein Querkörper (70) vorgesehen ist, der eine mit der Bohrung (4) korrespondierende Öffnung (71), zwei radial gegenüberliegende, mit dem Schlitz (7) korrespondierende Nocken (72) sowie einem radial von der Öffnung (71) sich wegerstreckenden Querfortsatz (73) zur Verbindung mit anderen Befestigungslementen (10) aufweist und welcher mittels einer Schraube (60) am oberen Abschnitt (3) befestigbar ist.

8. Befestigungselement nach einem der Ansprüche 1 - 7, dadurch gekennzeichnet, dass der Durchgangskanal (8) exzentrisch zur Längsachse (1) angeordnet ist und seitlich offen ist.

9. Befestigungselement nach einem der Ansprüche 1 - 7, dadurch gekennzeichnet, dass der Durchgangskanal (8) symmetrisch zur Längsachse (1) angeordnet ist gegen das obere Ende (6) des oberen Abschnittes (3) offen ist.

10. Osteosynthetisches Befestigungselement, insbesondere eine Pedikelschraube oder ein Wirbelsäulenhaken, mit einem Verschlussteil (30;50), mit einer Längsachse (1), mit einem am Knochen verankerbaren unteren Abschnitt (2) und einem in Richtung der Längsachse (1) daran anschliessenden, oberen Abschnitt (3), welcher an seinem unteren Ende von einem quer zur Längsachse (1) verlaufenden Durchgangskanal (8) zur Aufnahme eines Längsträgers (40) vollständig durchquert ist und welcher mit einem Innengewinde (5;31) und/oder einem Aussengewinde (9) versehen ist, um den den Längsträger (40) blockierenden Verschlussteil (30;50) aufzunehmen, wobei der obere Abschnitt (3) an seinem oberen Ende (6) oder an dem daran eingesetzten Verschlussteil (30;50) mit einem rotationsstabilen, lösbaren Verbindungsmechanismus (4,5,7;11,12) zur form- und kraftschlüssigen, temporären Aufnahme einer Manipulationsvorrichtung (20) versehen ist,
**dadurch gekennzeichnet, dass**
der obere Abschnitt (3) an seinem oberen Ende (6) oder das Verschlussteil (30;50) Querbohrungen (56) zur Aufnahme der Manipulationsvorrichtung mit entsprechenden Kugeln (55) aufweist.

11. Osteosynthetisches Befestigungselement, insbesondere eine Pedikelschraube oder ein Wirbelsäulenhaken, mit einem Verschlussteil (30;50), mit einer Längsachse (1), mit einem am Knochen verankerbaren unteren Abschnitt (2) und einem in Richtung der Längsachse (1) daran anschliessenden, oberen Abschnitt (3), welcher an seinem unteren Ende von einem quer zur Längsachse (1) verlaufenden Durchgangskanal (8) zur Aufnahme eines Längsträgers (40) vollständig durchquert ist und welcher mit einem Innengewinde (5;31) und/oder einem Aussengewinde (9) versehen ist, um den den Längsträger (40) blockierenden Verschlussteil (30;50) aufzunehmen, wobei der obere Abschnitt (3) an seinem oberen Ende (6) oder an dem daran eingesetzten Verschlussteil (30;50) mit einem rotationsstabilen, lösbaren Verbindungsmechanismus (4,5,7;11,12) zur form- und kraftschlüssigen, temporären Aufnahme einer Manipulationsvorrichtung (20) versehen ist,
**dadurch gekennzeichnet, dass**
der obere Abschnitt (3) an seinem oberen Ende (6) oder das Verschlussteil (30;50) im Kontaktbereich mit der Manipulationsvorrichtung (20) eine sich in Richtung der Längsachse (1) erstreckende polygonartige Bohrung aufweist und zur Aufnahme einer Kugel (55) der Manipulationsvorrichtung (20) einen rundsymmetrischen Hinterstich (62) aufweist.

12. Befestigungselement nach Anspruch 11, dadurch gekennzeichnet, dass die polygonartig ausgebildete Bohrung in den Flanken Teile eines zur Bohrung symmetrischen Innengewindes enthält.

13. Vorrichtung für Osteosynthesearbeiten mit einem Befestigungselement nach einem der Ansprüche 1-12 und mit einer ein mit dem Verbindungsmechanismus (4,5,7;11,12) des Befestigungselementes korrespondierendes Kupplungsteil (24,27;29) aufweisenden Manipuliervorrichtung.

14. Vorrichtung nach Anspruch 13, gekennzeichnet durch

   A) eine hohlzylindrische Hülse (21) mit der Längsachse (22), die an ihrem einen Ende (23) zwei quer zur Längsachse (22) verlaufende Nocken (24) aufweist; und
   B) einen vom anderen Ende (25) der Hülse (21) in letztere einführbaren zylindrischen Stift (26) mit einem, ein Aussengewinde (27) tragenden Ende, welches über das Ende (23) der Hülse (21) hervorragt und einem zur Handhabung dienenden aus der Hülse (21) vorstehenden Griffende (28).

15. Vorrichtung nach Anspruch 13 oder 14, dadurch gekennzeichnet, dass sie aus zwei miteinander gelenkig verbindbaren Teilen besteht, wovon der eine Teil (201) den Kupplungsteil (24,26,27) umfasst und der andere Teil (202) Bestandteil eines weiteren Instrumentes ist, vorzugsweise eine Zange zum Reponieren von Wirbeldeformitäten.

16. Vorrichtung nach Anspruch 15, dadurch gekennzeichnet, dass der eine Teil (201) einen kugelförmigen Teil (203) mit einem zylinderförmigen Fortsatz (204) aufweist, der mit einer entsprechenden seitlichen Öffnung (205) im anderen Teil (202) der Vorrichtung (20) korrespondiert.

17. Vorrichtung nach Anspruch 16, dadurch gekennzeichnet, dass der zylinderförmige Fortsatz (204) und/oder das Verbindungsstück (207) zum Teil (201) beidseitig abgeflacht sind, um eine Rotation des Befestigungselementes relativ zur Vorrichtung (20) um die Längsachse (1) des Befestigungselementes zu vermeiden.

18. Vorrichtung nach einem der Ansprüche 13 - 17, dadurch gekennzeichnet, dass sie einteilig ausgebildet ist, an dem, dem Befestigungselement zugekehrten Ende ein zum Innengewinde (5) entsprechendes Aussengewinde (27) aufweist, und im Zentrum eine Bohrung zur Aufnahme eines Dorns enthält.

19. Vorrichtung nach Anspruch 18, gekennzeichnet durch mindestens eine innerhalb des Kontaktbereiches mit dem Befestigungselement befindliche, mit Kugeln (55) versehene Querbohrung (56), wobei die Kugeln (55) durch einen in die Längsbohrung der Vorrichtung eingesetzten Dorn derart plaziert sind, dass sie aus der Querbohrung (56) hervorstehen, wobei der Überstand der Kugeln (55) kleiner als ihr Durchmesser ist.

## Claims

1. Osteosynthetic fixation element, particularly a pedicular screw or a vertebral column hook, having a lock part (30;50), with a longitudinal axis (1), with a lower section (2) that is anchorable at the bone and an upper section (3) connected to said lower section (2) in direction of said longitudinal axis (1), said upper section (3) being completely penetrated for acceptance of a longitudinal support (40) by a penetration channel (8) at its lower end, whereby said penetration channel (8) extends transversely to said longitudinal axis (1) and said upper section (3) being provided with an internal threading (5;31) and/or an external threading (9) to accept the lock part (30;50) locking the longitudinal support (40) whereby the upper section (3) is provided at its upper end (6) or at the inserted lock part (30;50) with a rotationally-stable, releasable connecting mechanism (4;5;7;11;12) for positiv and non-positiv temporary acceptance of a manipulation device (20),
characterized in that
the upper section (3) at its upper end (6) or the lock part (30;50) provides a slot (7) of depth $t_1$ running transversely to the longitudinal axis (1) for the acceptance of a manipulation device (20) with corresponding lugs (24).

2. Fixation element according to claim 1, characterized in that:

the upper section (3) provides a circular cylindrical drill hole (4) opening upwards, running in the direction of the longitudinal axis (1), with internal threading (5), whereby the drill hole (4) with its internal threading (5) extends from the floor of the slot (7) by the amount $t_2$ in the direction of the lower section (2), for the non-positive and/or positive acceptance of a manipulation device (20) with a corresponding external threading; and

the drill hole (4) is at least of the length $L = t_1 + t_2$ and permits a rotationally-stable, releasable connection with a manipulation device (20).

3. Fixation element according to claim 1, characterized in that the connecting mechanism of the upper section (3) consists of a bayonet connection.

4. Fixation element according to claim 3, characterized in that the bayonet connection consists of a slot (11) in the upper section (3) that preferably has a central drill hole and an internal threading.

5. Fixation element according to claim 2, characterized in that the upper section (3) is designed in two parts in such a manner that the connecting mechanism (4;5;7), consisting of the drill hole (4), internal threading (5) and slot (7) is realised as a lock part (30) shaped as a thread plug and screwable into the internal threading (31) of the upper section (3).

6. Fixation element according to one of the claims 1 - 5, characterized in that in addition a screw (60) with an external threading (61) which corresponds to the internal threading (5) is provided, which is screwable till into the range of the penetration channel (8), so that the longitudinal support (40) is fixable therewith.

7. Fixation element according to one of the claims 2, 5 or 6, characterized in that in addition a transverse body (70) is provided, that has an opening (71) corresponding to the drill hole (40), two radial opposite lugs (72) corresponding to the slot (7) and a transverse extension (73) extending radially from the opening (71) for connection with other fixation elements (10) and that can be fastened to the upper section (3) via a screw (60).

8. Fixation element according to one of the claims 1 - 7, characterised in that the penetration channel (8) is situated eccentrically to the longitudinal axis (1) and is open to the side.

9. Fixation element according to one of the claims 1 - 7, characterized in that the penetration channel (8) is placed symmetrically to the longitudinal axis (1) and is open at the upper end (6) of the upper section (3).

10. Osteosynthetic fixation element, particularly a pedicular screw or a vertebral column hook, having a lock part (30;50), with a longitudinal axis (1), with a lower section (2) that is anchorable at the bone and an upper section (3) connected to said lower section (2) in direction of said longitudinal axis (1), said upper section (3) being completely penetrated for acceptance of a longitudinal support (40) by a penetration channel (8) at its lower end, whereby said penetration channel (8) extends transversely to said longitudinal axis (1) and said upper section (3) being provided with an internal threading (5;31) and/or an external threading (9) to accept the lock part (30;50) locking the longitudinal support (40) whereby the upper section (3) is provided at its upper end (6) or at the inserted lock part (30;50) with a rotationally-stable, releasable connecting mechanism (4;5;7;11;12) for positiv and non-positiv temporary acceptance of a manipulation device (20), characterized in that the upper section (3) at its upper end (6) or the locking part (30;50) provides transverse drill holes (56) to the acceptance of the manipulation device with corresponding balls (55).

11. Osteosynthetic fixation element, particularly a pedicular screw or a vertebral column hook, having a lock part (30;50), with a longitudinal axis (1), with a lower section (2) that is anchorable at the bone and an upper section (3) connected to said lower section (2) in direction of said longitudinal axis (1), said upper section (3) being completely penetrated for acceptance of a longitudinal support (40) by a penetration channel (8) at its lower end, whereby said penetration channel (8) extends transversely to said longitudinal axis (1) and said upper section (3) being provided with an internal threading (5;31) and/or an external threading (9) to accept the lock part (30;50) locking the longitudinal support (40) whereby the upper section (3) is provided at its upper end (6) or at the inserted lock part (30;50) with a rotationally-stable, releasable connecting mechanism (4;5;7;11;12) for positiv and non-positiv temporary acceptance of a manipulation device (20), characterized in that the upper section (3) at its upper end (6) or the locking part (30;50) provides a polygon-like drill hole in the contact range with the manipulation device (20) extending in direction of the longitudinal axis (5) and provides a circular symmetrical recess (62) to the acceptance of a ball (55) of the manipulation device (20).

12. Fixation element according to claim 11, characterized in that the polygon-like shaped drill hole provides parts of a internal threading in its flanks which is symmetrical to the drill hole.

13. Device for osteosynthetic work with a fixation element according to one of the claims 1 - 12 and with a manipulation device providing a coupling part (24;27;29) corresponding to the connecting mechanism (4;5;7;11;12) of the fixation element.

14. Device according to claim 13, characterized in that

A) a hollow cylindrical sheath (21) with a longitudinal axis (22), which provides at its one end (23) two lugs (24) running transversely to the longitudinal axis (22); and
B) a cylindrical pin (26) which is insertable from the other end (25) of the sheath (21) into the latter, has an end bearing an external threading (27) that protrudes beyond the end (23) of the sheath (21) and a grip end (28) projecting from the sheath (21) that serves for manipulation.

15. Device according to one of the claims 13 or 14, characterized in that it consists of two parts joinable connected to each other, of which the one part (201) comprises the coupling part (24;26;27) and the other part (202) is a component of a further instrument, preferably pincers for repositioning vertebral deformities.

16. Device according to claim 15, characterized in that the one part (201) provides a ball-shaped part (203) with a cylindrical extension (204) that corresponds to a corresponding lateral opening (206) in the other part (202) of the device (20).

17. Device according to claim 16, characterized in that the cylindrical extension (204) and/or the connecting part (207) is bilaterally flattened at the part (201) to avoid rotation of the fixation element relative to the device (20) around the longitudinal axis (1) of the fixation element.

18. Device according to one of the claims 13 - 17, characterized in that it is formed in one piece, provides an external threading (27) corresponding to the internal threading and situated at the end turned towards the fixation element and contains in the center a drill hole for the acceptance of a pin.

19. Device according to claim 19, characterized by at least one transverse drill hole (56) provided with balls (55), located within the contact area of the fixation element, where the balls (55) are situated in such a manner by means of a pin inserted in the longitudinal drill hole of the device that they protrude from the transverse drill hole (56) whereby the overhang of the balls (55) is less than their diameter.

**Revendications**

1. Elément de fixation pour ostéosynthèse, en particulier vis à pédicule ou crochet pour colonne vertébrale, comportant une pièce de verrouillage (30; 50) à axe longitudinal (1), une partie inférieure (2) qui peut être ancrée dans l'os et une partie supérieure (3) qui s'y raccorde en direction de l'axe longitudinal (1) et qui, à son extrémité inférieure, est complètement traversée par un canal de traversée (8) qui s'étend transversalement par rapport à l'axe longitudinal (1) et sert à recevoir un support longitudinal (40), et qui est dotée d'un filet interne (5; 31) et/ou d'un filet externe (9) pour recevoir la pièce de verrouillage (30; 50) qui bloque le support longitudinal (40), la partie supérieure (3) étant dotée à son extrémité supérieure (6) ou sur la pièce de verrouillage (30; 50) qui y est insérée, d'un mécanisme de liaison amovible (4, 5, 7; 11, 12), à rotation solidaire, pour la réception temporaire, en correspondance géométrique et mécanique, d'un dispositif de manipulation (20),
caractérisé en ce que la partie supérieure (3) présente, à son extrémité supérieure (6) ou sur la pièce de verrouillage (30; 50), une fente (7) s'étendant transversalement par rapport à l'axe longitudinal (1), de profondeur $t_1$, pour la réception du dispositif de manipulation (20) doté de cames correspondantes (24).

2. Elément de fixation selon la revendication 1, caractérisé en ce que la partie supérieure (3) présente un alésage (4) doté d'un filet interne (5), ouvert dans le haut, cylindrique circulaire, s'étendant dans la direction de l'axe longitudinal (1), l'alésage (4) s'avançant par son filet interne (5) depuis le fond de la fente (7) sur la distance $t_2$ en direction de la partie inférieure (2), pour la réception en correspondance mécanique et/ou géométrique d'un dispositif de manipulation (20) doté d'un filet externe correspondant (27); et
l'alésage (4) présente au moins la longueur $L = t_1 + t_2$ et permet une liaison libérable, à rotation solidaire, à un dispositif de manipulation (20).

3. Elément de fixation selon la revendication 1, caractérisé en ce que le mécanisme de liaison de la partie supérieure (3) est constitué d'un verrou à baïonnette.

4. Elément de fixation selon la revendication 3, caractérisé en ce que le verrou à baïonnette est constitué d'une fente (11) dans la partie supérieure (3), qui présente de préférence un alésage central à filet interne.

5. Elément de fixation selon la revendication 2, caractérisé en ce que la partie supérieure (3) est réalisée en deux pièces, de telle sorte le mécanisme de liaison (4, 5, 7), constitué de l'alésage (4), du filet interne (5) et de la fente (7), est réalisé sous la forme d'une pièce de verrouillage (30) configurée comme bouchon fileté pouvant être vissé dans le filet interne (31) de la partie supérieure (3).

6. Elément de fixation selon l'une des revendications 1 à 5, caractérisé en ce qu'il y est en outre prévu une vis (60) dotée d'un filet externe (61) correspondant au filet interne (5), et qui peut être vissée jusque dans la région du canal de traversée (8), de manière à pouvoir fixer le support longitudinal (40).

7. Elément de fixation selon l'une des revendications 2, 5 ou 6, caractérisé en ce qu'il y est en outre prévu un corps transversal (70) qui présente une ouverture (71) correspondant à l'alésage (4), deux cames radialement opposées (72), correspondant à la fente (7), ainsi qu'un appendice transversal (73) s'éloignant radialement de l'ouverture (71), pour la liaison à d'autres élément de fixation (10), et qui peut être fixé sur la partie supérieure (3) au moyen d'une vis (60).

8. Elément de fixation selon l'une des revendications 1 à 7, caractérisé en ce que le canal de traversée (8) est disposé en position décentrée par rapport à l'axe longitudinal (1) et est ouvert latéralement.

9. Elément de fixation selon l'une des revendications 1 à 7, caractérisé en ce que le canal de traversée (8) est disposé symétriquement par rapport à l'axe longitudinal (1), et est ouvert en direction de l'extrémité supérieure (6) de la partie supérieure (3).

10. Elément de fixation pour ostéosynthèse, en particulier vis à pédicule ou crochet pour colonne vertébrale, comportant une pièce de verrouillage (30; 50), un axe longitudinal (1), une partie inférieure (2) qui peut être ancrée dans l'os et une partie supérieure (3) qui s'y raccorde en direction de l'axe longitudinal (1), qui à son extrémité inférieure est traversée complètement par un canal de traversée (8) s'étendant transversalement par rapport à l'axe longitudinal (1), pour la réception d'un support longitudinal (40), et qui est dotée d'un filet interne (5; 31) et/ou d'un filet externe (9) pour recevoir la pièce de verrouillage (30; 50) bloquant le support longitudinal (40), la partie supérieure (3 étant dotée à son extrémité supérieure (6) ou sur la pièce de verrouillage (30; 50) insérée dans celle-ci d'un mécanisme de liaison libérable (4, 5, 7; 11, 12), à rotation solidaire, pour la réception temporaire en correspondance géométrique et mécanique d'un dispositif de manipulation (20),

caractérisé en ce que
la partie supérieure (3) présente à son extrémité supérieure (6) ou sur la pièce de verrouillage (30; 50) des alésages transversaux (56) pour la réception du dispositif de manipulation, avec billes correspondantes (55).

11. Elément de fixation pour ostéosynthèse, en particulier vis à pédicule ou crochet pour colonne vertébrale, comportant une pièce de verrouillage (30; 50), un axe longitudinal (1), une partie inférieure (2) qui peut être ancrée dans l'os et une partie supérieure (3) qui s'y raccorde en direction de l'axe longitudinal (1), qui à son extrémité inférieure est traversée complètement par un canal de traversée (8) s'étendant transversalement par rapport à l'axe longitudinal (1), pour la réception d'un support longitudinal (40), et qui est dotée d'un filet interne (5; 31) et/ou d'un filet externe (9) pour recevoir la pièce de verrouillage (30; 50) bloquant le support longitudinal (40), la partie supérieure (3 étant dotée à son extrémité supérieure (6) ou sur la pièce de verrouillage (30; 50) insérée dans celle-ci d'un mécanisme de liaison libérable (4, 5, 7; 11, 12), à rotation solidaire, pour la réception temporaire en correspondance géométrique et mécanique d'un dispositif de manipulation (20),
caractérisé en ce que
la partie supérieure (3) présente à son extrémité supérieure (6) ou la pièce de verrouillage (30; 50) présente dans la région de contact avec le dispositif de manipulation (20), un alésage polygonal s'étendant dans la direction de l'axe longitudinal (1) et une contre - dépouille (62) à symétrie circulaire pour la réception d'une bille (55) du dispositif de manipulation (20).

12. Elément de fixation selon la revendication 11, caractérisé en ce que l'alésage de forme polygonale contient dans ses flancs des parties d'un filet interne symétriques par rapport à l'alésage.

13. Dispositif pour travaux d'ostéosynthèse, comportant un élément de fixation selon l'une des revendications 1 à 12, et comportant un dispositif de manipulation présentant une pièce d'accouplement (24, 27, 29) correspondant au mécanisme de liaison (4, 5, 7; 11, 12) de l'élément de fixation.

14. Dispositif selon la revendication 13, caractérisé par

    A) une douille cylindrique creuse (21) d'axe longitudinal (22), qui présente à l'une de ses extrémité (23) deux cames (24) s'étendant transversalement par rapport à l'axe longitudinal (22); et
    B) une tige cylindrique (26) qui peut être insérée dans la douille (21) par l'autre extrémité (25) de celle-ci, comportant une extrémité portant un filet externe (27), qui déborde au-delà de l'extrémité (23) de la douille (21) et une extrémité de poignée (28) en saillie hors de la douille (21) et servant à la manipulation de déformations de la colonne vertébrale.

15. Dispositif selon les revendications 13 ou 14, caractérisé en ce qu'il est constitué de deux parties qui peuvent être reliées l'une à l'autre de manière articulée, dont une partie (201) comporte la pièce d'accouplement (24, 26, 27) et l'autre partie (202) fait partie d'un autre instrument, de préférence une pince pour le redressement de déformations.

16. Dispositif selon la revendication 15, caractérisé en ce que la partie (201) présente une pièce (203) en forme de bille comportant un appendice cylindrique (204) qui correspond à une ouverture latérale (205) correspondante dans l'autre partie (202) du dispositif (20).

17. Dispositif selon la revendication 16, caractérisé en ce que l'appendice cylindrique (204) et/ou la pièce de liaison (207) sont aplatis des deux côtés vers la partie (201), pour empêcher une rotation de l'élément de fixation par rapport au dispositif (20) autour de l'axe longitudinal (1) de l'élément de fixation.

18. Dispositif selon l'une des revendications 13 à 17, caractérisé en ce qu'il est réalisé en une seule pièce, dont l'extrémité tournée vers l'élément de fixation présente un filet externe (27) correspondant au filet interne (5), et qui contient au centre un alésage pour la réception d'une broche.

19. Dispositif selon la revendication 18, caractérisé par au moins un alésage transversal (56), doté de billes (55), situées à l'intérieur de la région du contact avec élément de fixation, les billes (55) étant déplacées par une broche insérée dans l'alésage longitudinal du dispositif de telle sorte qu'elles débordent hors de l'alésage transversal (56), la partie débordante des billes (55) étant plus petite que leur diamètre.

Fig. 1

Fig. 2

Fig. 3

Fig. 4

Fig. 5

Fig. 6

Fig. 7

Fig. 8

Fig. 9

Fig. 11

Fig. 10

Fig. 12

## Fig. 13

## Fig. 14